Europäisches Patentamt

European Patent Office

Office européen des brevets

(11) Publication number: 0 420 502 B1

## EUROPEAN PATENT SPECIFICATION

(45) Date of publication of patent specification: 25.05.94

(51) Int. Cl.5: **C12N 15/58**, C12N 9/64, C12N 5/10

(21) Application number: 90310305.9

(22) Date of filing: 20.09.90

(54) A new plasminogen activator derivative and a method for its manufacture.

(30) Priority: 20.09.89 JP 243750/89
17.10.89 JP 269406/89
01.03.90 JP 50428/90
02.08.90 JP 206458/90

(43) Date of publication of application:
03.04.91 Bulletin 91/14

(45) Publication of the grant of the patent:
25.05.94 Bulletin 94/21

(84) Designated Contracting States:
BE DE FR GB IT

(56) References cited:
WO-A-87/04722

THE JOURNAL OF BIOLOGICAL CHEMISTRY,
vol. 265, no. 10, 5th April 1990, pages
5540-5545, The American Society for Bio-
chemistry and Molecular, Inc.; T.J.AHREN et
al.: "Site-directed mutagenesis in human tis-
sue-plasminogen activator"

(73) Proprietor: KANEGAFUCHI KAGAKU KOGYO
KABUSHIKI KAISHA
2-4 Nakanoshima 3-chome
Kita-ku Osaka-shi Osaka-fu 530(JP)

(72) Inventor: Yahara, Hitoshi
830-161, Ikeda, Onoe-cho
Kakogawa-shi, Hyogo-ken(JP)
Inventor: Nagaoka, Tetsuya
2-63, Okihama-machi, Takasago-cho
Takasago-shi, Hyogo-ken(JP)
Inventor: Yajima, Kazuyoshi
1-413-305, Nakaochiai 1-chome, Suma-ku
Kobe-shi, Hyogo-ken(JP)
Inventor: Ikenaka, Yasuhiro
610-1-404, Nishijima, Okubo-cho
Akashi-shi, Hyogo-ken(JP)
Inventor: Matsumoto, Keiji
20-16-204, Tendo-cho
Nishinomiya-shi, Hyogo-ken(JP)
Inventor: Kakutani, Tetsu
90-43, Shinnobe, Befu-cho
Kakogawa-shi, Hyogo-ken(JP)

THE JOURNAL OF BIOLOGICAL CHEMISTRY, vol. 265, no. 21, 25th July 1990, pages 12184-12191, Baltimore, US; D. COLLEN et al.: "Biochemical and functional characterization of human tissue-type plasminogen activator variants with mutagenized kringle domains"

(74) Representative: **Nash, David Allan et al**
**Haseltine Lake & Co.**
**Hazlitt House**
**28 Southampton Buildings**
**Chancery Lane**
**London WC2A 1AT (GB)**

## Description

The present invention relates to a novel derivative of TPA which is useful in the treatment of thrombosis; a DNA sequence encoding the said derivative; an expression vector comprising the said DNA sequence; a transformant into which the said vector has been introduced; and a method for producing the said derivative.

Concerning human TPA (hereinafter TPA is referred to as TPA), particularly detailed research has been devoted to TPA secreted by human melanoma (i.e., Bows melanoma) cells, and TPA is known to be a glyco-protein comprising 572 amino acid residues [Pennica, D., et al., Nature, 301, 214 (1983)].

TPA is an enzyme which acts upon the plasminogen which is present in the blood and converts this plasminogen into plasmin. The plasmin dissolves the fibrin which causes thrombi. TPA possesses strong affinity with fibrin, moreover, the activity of TPA is fibrin-dependent, hence, TPA is regarded as acting specifically upon thrombi. Therefore, TPA is used therapeutically for the treatment of various types of thrombosis [Crossbard, E B., Pharmaceutical Research, 4, 375 (1987)]. However, when TPA is administered into the blood stream for the treatment of thrombosis, long-sustained effect is not obtained, and the TPA rapidly vanishes from the blood stream, which constitutes a problem in therapeutic applications. TPA is believed to be metabolized principally in the liver [Fuchs, H. E., et al., Blood, 65, 539 (1985)]. The half-life of TPA in the blood is only about 2 minutes [Collen, D., et al., Circulation, 72, 384 (1985)]. Consequently, large quantities of TPA must be administered in order to maintain a constant effective concentration of TPA in the blood. Moreover, since the solubility of TPA in water is low, administration at high concentrations is impossible, and therefore the administration of the prescribed quantity of TPA requires considerable time. Thus, in order to achieve effective treatment of thrombosis, large quantities of TPA must be administered over an extended period of time, which renders TPA impractical for the treatment of urgent cases of thrombosis. Furthermore, being a protein which occurs only in minute quantities in nature, TPA is expensive, consequently to administrate large doses of TPA is extremely expensive and imposes a great economic burden upon the patient.

With a view to solving these problems, various TPA derivatives, with improved persistence in the blood obtained by chemical or enzymatic modification or by genetic engineering methods, have been reported in the literature [Browne, M. J., et al., J Biol. Chem., 263, 1599 (1988); Dodd, I., et al., Thrombosis and Haemostasis, 59, 523 (1988); Kayan, N. K., et al., J Biol. Chem., 263, 3971 (1988)]. However, although greatly improved with respect to persistence in the blood, the TPA derivatives described in these reports have proved to be extremely inferior to the native TPA with regard to the affinity with fibrin that is one of the characteristic properties of TPA [Larsen, G. R., et al., J. Biol. Chem., 263, 1023 (1988)]. Furthermore, in some case, these TPA derivatives also display a pronounced decrease in fibrinolytic ability [Hansen, L., et al., J. Biol. Chem., 263, 15713 (1988)].

Thus, TPA derivatives with superior therapeutic efficacy have not yet been obtained. Therefore, there has existed a need for the development of TPA derivatives which permit effective treatment of thrombosis with small doses by virtue of improved persistence in the blood; retain the original biochemical characteristics of TPA to the greatest possible extent; and cause no significant increases in hemorrhagic or other adverse side effects.

TPA is composed of five regions, i.e., a finger region, a growth factor region, a kringle 1 region, a kringle 2 region, and a region possessing serine protease activity. These regions are aligned in the above-mentioned order from the N-terminus of the amino acid sequence of TPA (Pennica, D., et. al., Nature, 301, 214, supra). All of the TPA derivatives created thus for and evaluated the ability of persistence in the blood and fibrinolytic ability, lack the finger region alone, or lack an extensive region including finger region and other regions [Hansen, L., et al., J. Biol. Chem., 263, 15713, supra; Larsen, G. R., et al., J. Biol. Chem., 263, 1023, supra; and Collen, D., et al., Blood, 71, 219 (1988)]. These types of TPA derivatives, lacking the finger region, are improved with respect to persistence in the blood, but display markedly decreased thrombolytic ability. Therefore, plasminogen activation and fibrinolytic properties are believed to arise from the finger region.

The aforesaid kringle regions have also been studied. The kringle 2 region is said to be concerned in the affinity of TPA for fibrin and the property of activation of TPA by fibrin. On the other hand, although derivatives lacking the kringle 1 region have been created and studied, the function of this region has not yet been adequately clarified [Zonneveld, A. J. V., et al., Journal of Biological Chemistry, 261, 14214 (1986); Proc. N. A. S., 83, 169 (1986)].

The present inventors endeavored to create TPA derivatives characterized by long persistence in the blood and high affinity for plasminogen by appropriately modifying the finger region without loss of the essential characteristics of this region, or by modifying the kringle regions, or by modifying both the finger and kringle regions. Moreover, with the objective of enhancing the affinity for fibrin and the property of activation by fibrin which are possessed by the kringle 2 region, the amino acid sequence of the kringle 1 region was converted into a sequence resembling the kringle 2 region. In this manner, using genetic engineering methods, a large number of amino acid substitution products were created. As a result, among a number of TPA derivatives with the 37th through 42nd amino acids included in the finger region replaced by other amino acids, especially by hydrophobic amino acids, and/or the amino acids of a portion of the kringle 1 region replaced by other prescribed amino acids, certain particular TPA derivatives were found to possess the same degree of thrombolytic effectiveness as native TPA, and, moreover, to display greatly extended lifetimes in the blood, thereby realizing the completion of the present invention.

WO 87/04722 discloses thrombolytic proteins which have tissue plasminogen-type activity and which are characterized by modification within the 94 amino acid N-terminus and/or at Arg-275 and/or at one of the N-linked glycosylation sites

According to the present invention, there is provided a tissue plasminogen activator derivative, characterized by a greatly extended persistence in blood as compared with native TPA, comprising;

(i) asparagine at the 37th position being replaced by serine,

(ii) serine at the 38th position being replaced by valine,

(iii) glycine at the 39th position being replaced by valine,

(iv) arginine at the 40th position being replaced by glutamic acid or isoleucine,

(v) alanine at the 41st position being replaced by serine, phenylalanine, or valine, and

(vi) glutamine at the 42nd position being replaced by serine,

and / or further comprising;

(vii) glycine at the 161st position being replaced by arginine,

(viii) lysine at the 162nd position being replaced by arginine, and

(ix) serine at the 165th position being replaced by tryptophan,

wherein said position number is determined from the N-terminus of the amino acid sequence of natural tissue plasminogen activator.

According to the present invention, there is also provided a tissue plasminogen activator derivative, characterized by a greatly extended persistence in blood as compared with native TPA, comprising;

(i) asparagine at the 37th position being replaced by serine,

(ii) serine at the 38th position being replaced by valine,

(iii) glycine at the 39th position being replaced by valine,

(iv) arginine at the 40th position being replaced by glutamic acid or isoleucine,

(v) alanine at the 41st position being replaced by serine, phenylalanine, or valine, and

(vi) glutamine at the 42nd position being replaced by serine,

and

(vii) asparagine at the 115th position which is in the kringle 1 domain being replaced by proline,

wherein said position number is determined from the N-terminus of the amino acid sequence of natural tissue plasminogen activator.

The DNA sequence of this invention encodes the above-mentioned TPA derivative.

In a preferred embodiment, the DNA sequence is derived from cDNA or genomic DNA.

The expression vector of this invention comprises the above-mentioned DNA sequence.

The transformant of this invention is obtained by introducing the above-mentioned expression vector into a cultured animal cell.

The glycosylated TPA derivative of this invention is obtained by cultivating the transformant.

The method for producing a TPA of this invention comprises the steps of:

(a) constructing an expression vector which comprises the above-mentioned DNA sequence,

(b) introducing said expression vector into a cultured animal cell, resulting in a transformant,

(c) cultivating said transformant to produce a TPA derivative, and

(d) recovering the produced TPA derivative.

Thus, the invention described herein makes possible the objectives of, (1) providing a novel TPA derivative characterized by long half-life in the blood and high fibrinolytic ability; (2) providing a novel TPA derivative which can be effectively used for the treatment of thrombotic disorders such as myocardial infarction by virtue of far improved persistence in the blood as compared with native TPA as well as retention of the same level of thrombolytic ability as native TPA; (3) providing a novel high-potency TPA derivative which permit the improvement of the therapeutic efficacy of TPA treatment now being undertaken in connection with various vascular disorders; (4) providing a DNA sequence encoding the TPA derivative possessing the aforesaid superior properties, an expression vector containing the said DNA sequence, and a transformant obtained by the introduction of the said expression vectors; and (5) providing a method for producing a TPA derivative by cultivation of the said transformant.

For a better understanding of the invention and to show how the same may be carried into effect reference will now be made, by way of example only, to the accompanying drawings, in which:

Figure 1 is a schematic diagram showing the construction of the TPA expression vector pSVeCPA-1 used for the construction of expression vectors of the present invention.

Figure 2 is a schematic diagram showing the construction of the vector pSVePN1 which is one of the expression vectors of the present invention.

Figure 3 is a schematic diagram showing the construction of the vector pSVeDS1 which is one of the expression vectors of the present invention.

Figure 4 is a schematic diagram showing the construction of the vector pSVeSD8 which is one of the expression vectors of the present invention.

Figure 5 is a schematic diagram showing the construction of the vector pSVeKS48 which is one of the expression vectors of the present invention.

Figure 6 is a schematic diagram showing the amino acid sequence of the finger region in the neighborhood of the N-terminus of TPA as well as the amino acid sequences of the TPA derivatives PN1, PN2, PN3 and DS1 in the finger region.

Figure 7 shows the results of the evaluation of in vitro thrombolytic ability of TPA derivatives SD4, SD8, and KS48 of the present invention.

Figure 8 shows the in vivo clearance of TPA derivatives PN1, PN2, PN3, DS1, and SD1 of the present invention in rabbit blood.

Figure 9 shows the in vivo clearance of TPA derivatives KM4, KS48, SD4, SD8, and KS218 of the present invention in rabbit blood.

In the following account, the present invention will be described in accordance with the order of the processes employed for the production of TPA derivatives of the present invention.

(I) Preparation of a DNA sequence encoding TPA

In order to modify the finger region and/or kringle region of TPA by genetic engineering techniques, and thereby create TPA derivatives with improved blood persistence, a DNA sequence encoding the amino acid sequence of native TPA is required. Such a DNA sequence can be obtained by cloning cDNA or genomic DNA (hereinafter, genomic DNA is referred to as gDNA), or by chemical synthesis of DNA on the basis of the appropriate cDNA, gDNA, or TPA amino acid sequences.

cDNA encoding TPA has already been isolated by [Pennica, D. et al. (Nature, 301, 214 (1983), supra], while gDNA encoding TPA has been isolated by Ny, T. et al. [Proc. Natl. Acad. Sci. USA, 81, 5355 (1984)]; Brown, M. J. et al. [Gene, 33, 279 (1985)]; and Degen, S. J. F. et al. [J. Biol. Chem., 261, 6972 (1986)]. In the specification, the numbering of amino acid and cDNA sequences of TPA is in accordance with that of Pennica, D. et al. (supra), while the numbering of exons follows that of Ny, T. et al. (supra).

In addition, various TPA expression vectors obtained by gene manipulation are known, and, for example, a DNA sequence can be obtained by utilizing the TPA expression vector pSVePA-1 described in Japanese Laid-Open Patent Publication No. 62-14783. An example of the construction of an expression vector for a TPA derivative of the present invention is as follows.

First, mRNA is isolated from a host containing the aforesaid expression vector pSVePA-1, for example, CHO-K1 cells transformed by this vector (Japanese Laid-Open Patent Publication No. 62-14783, supra). Then, cDNA is synthesized from this mRNA, a cDNA library is constructed, and cloning is performed. Cloning can be performed, for example, by plaque hybridization, using as a probe, a DNA fragment (encoding TPA) obtained by cleavage of the aforesaid expression vector with an appropriate restriction enzyme. In this manner, the clone CH79 which displays positive hybridization with this probe can be selected. When the DNA in this clone CH79 is isolated and the DNA sequence is analyzed by the Southern hybridization method, it is found that this clone contains a DNA fragment which hybridizes with the aforesaid probe and which can be cleaved to approximately 2.2 kb by HindIII. The CDNA clone pCH7g is then obtained by inserting this HindIII-cleaved fragment into a plasmid vector pUC19. Determination of the base sequence of the cDNA portion of this clone pCH79 by the M13 method reveals that the 5′ terminus of this cDNA portion is linked with a HindIII recognition site originating from the plasmid vector pUC19, while approximately 150 bp downstream from this a BglII recognition site is present, and the stop codon TGA is located approximately 1500 bp further downstream. About 410 bp downstream from this TGA codon, an another HindIII recognition site originating from the plasmid vector pUC19 is present. Except for the substitution of thymine for cytosine at the 585th position and cytosine for adenine at the 1725th position, the base sequence of this cDNA portion is identical with that of the base sequence of TPA as reported by Pennica D. et al. (Nature, 301, 234, supra).

Using the cDNA sequence encoding TPA obtained in this manner, the TPA expression vectors which are essential for the preparation of the TPA derivatives can be constructed.

(II) Construction of expression vectors

The construction of natural-type, TPA expression vector pSVeCPA-1 and TPA derivative expression vectors is described as follows.

(II-A) Construction of natural-type TPA expression vector pSVeCPA-1

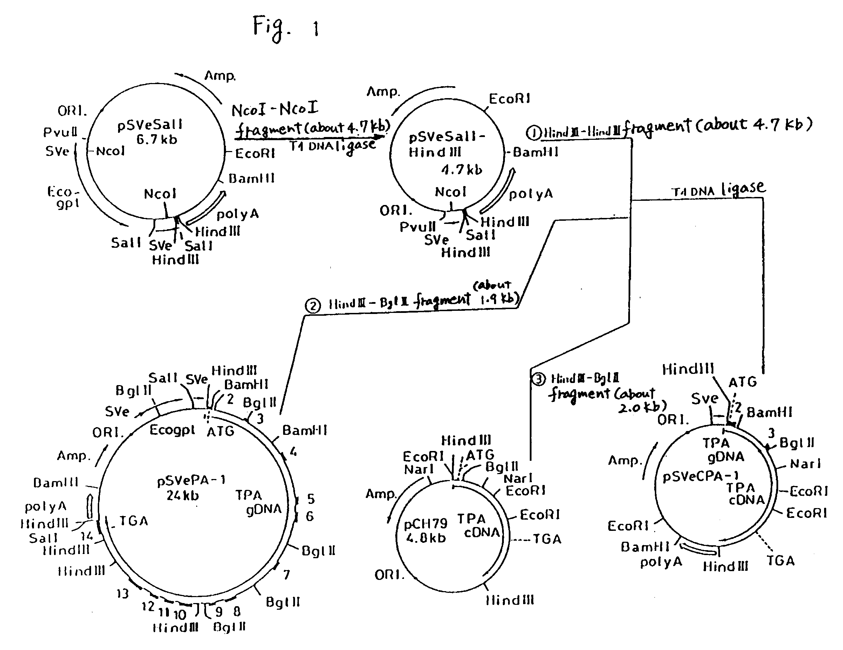

A natural-type TPA expression vector pSVeCPA-1 can be constructed by using the plasmid vector pSVeSal I (Japanese Laid-open Patent Publication No. 62-14783, supra), the gDNA contained in the aforesaid vector pSVePA-1, and the cDNA of the aforesaid TPA. Specifically, the TPA expression vector pSVeCPA-1 is constructed by ligating the following three DNA fragments 1, 2, and 3. The construction of this expression vector is schematically shown in Figure 1.

(1) HindIII-HindIII fragment of pSVeSal I

The plasmid vector pSVeSal I (Japanese Laid-Open Patent Publication No. 62-14783, supra) is cleaved with a restriction enzyme NcoI, the larger fragment (NcoI-NcoI fragment, approximately 4.7 kb) is isolated, and circularized with T4 DNA ligase. The plasmid vector pSVeSal I-HindIII so obtained is cleaved with HindIII, and the larger of the resulting DNA fragments (approximately 4.7 kb) is recovered.

(2) HindIII-BglII fragment of pSVePA-1

The TPA expression vector pSVePA-1 constructed by the use of the aforesaid gDNA is cleaved with HindIII and BglII, and the smaller of the resulting DNA fragments (approximately 1.9 kb) is recovered.

(3) BglII-HindIII fragment of pCH79

This DNA fragment (approximately 2 kb), containing cDNA of TPA, is obtained by cleavage of the aforesaid cDNA clone pCH79 with BglII and HindIII.

The TPA expression vector pSVeCPA-1 constructed in this manner is designed so that the early promoter of the SV40 virus is present upstream from the TPA gene in a position and direction permitting expression of the TPA gene, and TPA can be produced when the vector is introduced into mammalian cells. Not only the SV40 promoter but in fact any promoter which permits the expression of the TPA gene can be used for this purpose.

(II-B) Construction of an expression vector for TPA derivative with a modified finger region

Methods of preparing an amino acid-substituted variant of TPA (i.e., TPA derivative), with certain amino acid residues in the finger region replaced by different amino acids, include the following.
(1) Using a synthetic DNA primer to introduce mutations into a DNA sequence encoding the amino acid sequence of TPA.
(2) Introducing mutations in cassette form by direct use of synthetic DNA.
If the above method 2 is applied to cDNA of TPA, the synthesized DNA sequence may be either the entire cDNA or merely an appropriate portion (i.e., the portion into which mutations are introduced). If only a portion is synthesized, then, by using a suitable restriction endonuclease (i.e., restriction enzyme and T4 DNA ligase), a portion of the original cDNA sequence can be replaced by the synthetic DNA. For example, the following twelve varieties of TPA derivatives with amino acid replacements to be described below, i.e., PN1, PN2, PN3, DS1, SD1, SD2, SD3, SD4, SD5, SD6, SD7 and SD8, can be produced by synthesizing DNA sequences encoding portions of an amino acid sequence including the amino acid replacements and constructing expression vectors containing the cDNA sequence of native TPA with the corresponding portions replaced by the said synthetic DNA sequences.
PN1: a TPA derivative in which arginine at the 7th position, aspartic acid at the 8th position, and gultamic acid at the 9th position from the N-terminus of the amino acid sequence of natural TPA are replaced by lysines, respectively.

EP 0 420 502 B1

PN2: a TPA derivative in which lysine at the 10th position, threonine at the 11th position, glutamine at the 12th position, methionine at the 13th position, and isoleucine at the 14th position from the N-terminus of the amino acid sequence of natural TPA are replaced by glutamic acid, valine, serine, serine, and serine, respectively.

PN3: a TPA derivative in which tyrosine at the 15th position, glutamine at the 16th position, glutamine at the 17th position, histidine at the 18th position, and glutamine at the 19th position from the N-terminus of the amino acid sequence of natural tissue plaminogen activator are replaced by phenylalanine, serine, serine, glutamic acid, and serine, respectively.

DS1: a TPA derivative in which serine at the 28th position, asparagine at the 29th position, arginine at the 30th position, valine at the 31st position, and glutamic acid at the 32nd position, and tyrosine at the 33rd position from the N-terminus of the amino acid sequence of natural TPA are replaced by valine, serine, glutamic acid, serine, lysine, and asparginine, respectively.

SD1: a TPA derivative in which asparagine at the 37th position, serine at the 38th position, glycine at the 39th position, arginine at the 40th position alanine at the 41st position and glutamine at the 42nd position from the N-terminus of the amino acid sequence of natural TPA are replaced by serine, valine, valine, gulutamic acid, serine, and serine, respectively.

SD2: a TPA derivative in which asparagine at the 37th position, serine at the 38th position, glycine at the 39th position, argininen at the 40th position alanine at the 41st position and glutamine at the 42nd position from the N-terminus of the amino acid sequence of natural TPA are replaced by leucine, valine, valine, isoleucine, valine, and leucine, respectively.

SD3: a TPA derivative in which asparagine at the 37th position, serine at the 38th position, glycine at the 39th position, arginine at the 40th position alanine at the 41st position and glutamine at the 42nd position from the N-terminus of the amino acid sequence of natural TPA are replaced by serine, valine, valine, isoleucine, serine, and serine, respectively.

SD4: a TPA derivative in which asparagine at the 37th position, serine at the 38th position, glycine at the 39th position, arginine at the 40th position alanine at the 41st position and glutamine at the 42nd position from the N-terminus of the amino acid sequence of natural TPA are replaced by serine, valine, valine, isoleucine, valine, and serine, respectively.

SD5: a TPA derivative in which asparagine at the 37th position, serine at the 38th position, glycine at the 39th position, argininen at the 40th position alanine at the 41st position and glutamine at the 42nd position from the N-terminus of the amino acid sequence of natural TPA are replaced by serine, valine, valine, isoleucine, valine, and leucine, respectively.

SD6: a TPA derivative in which asparagine at the 37th position, serine at the 38th position, glycine at the 39th position, argininen at the 40th position alanine at the 41st position and glutamine at the 42nd position from the N-terminus of the amino acid sequence of natural TPA are replaced by phenylalanine, leucine, phenylalanine, valine, leucine, and phenylalanine, respectively.

SD7: a TPA derivative in which asparagine at the 37th position, serine at the 38th position, glycine at the 39th position, arginine at the 40th position alanine at the 41st position and glutamine at the 42nd position from the N-terminus of the amino acid sequence of natural TPA are replaced by valine, phenylalanine, leucine, phenylalanine, isoleucine, and valine, respectively.

SD8: a TPA derivative in which asparagine at the 37th position, serine at the 38th position, glycine at the 39th position, arginine at the 40th position alanine at the 41st position and glutamine at the 42nd position from the N-terminus of the amino acid sequence of natural TPA are replaced by serine, valine, valine, glutamic acid, phenylalanine, serine, respectively.

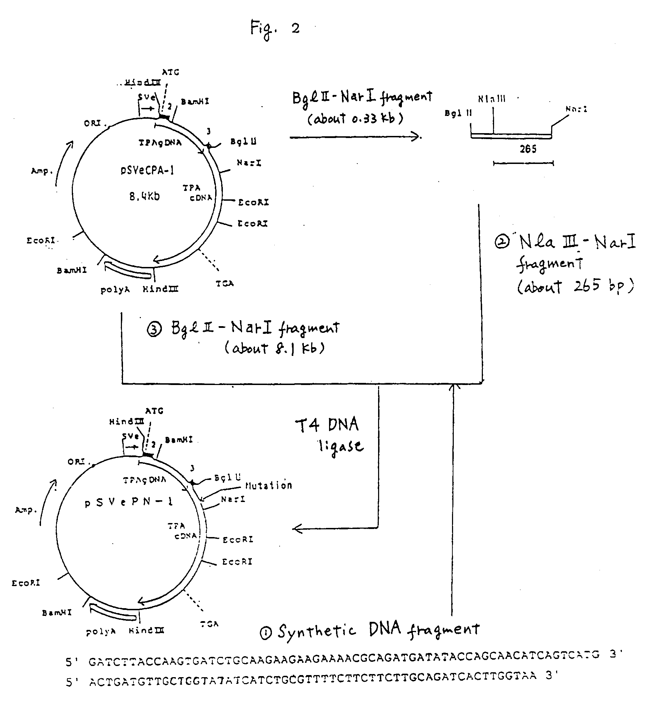

The expression vectors pSVePN1, pSVePN2 and pSVePN3 used for the production of the aforesaid TPA derivatives PN1, PN2 and PN3, respectively, can be constructed by ligating the following three types of DNA fragments 1, 2, and 3 by the use T4 DNA ligase. The construction of these expression vectors is schematically illustrated in Figure 2, employing pSVePN1 as a typical example.

(1) Synthetic DNA fragments

Single-stranded synthetic DNA fragments with the following DNA sequences, two varieties for each vector, are synthesized. The two varieties of single-stranded DNA for each vector are complementary, and prior to the construction of the expression vector are annealed by a conventional method for use in the form of double-stranded DNA.

Synthetic DNA fragments for construction of pSVePN1

5' GATCTTACCAAGTGATCTGCAAGAAGAAGAAAACGCAGATGATATACCAGCAACATCAGTCATG '3
5' ACTGATGTTGCTGGTATATCATCTGCGTTTTCTTCTTCTTGCAGATCACTTGGTAA 3'

Synthetic DNA fragments for construction of pSVePN2

5' GATCTTACCAAGTGATCTGCAGAGATGAAGAGGTCTCCTCCTCCTACCAGCAACATCAGTCATG 3'
5' ACTGATGTTGCTGGTAGGAGGAGGAGACCTCTTCATCTCTGCAGATCACTTGGTAA 3'

Synthetic DNA fragments for construction of pSVePN3

5' GATCTTACCAAGTGATCTGCAGAGATGAAAAAACGCAGATGATATTCTCCTCTGAGTCCTCATG 3'
5' AGGACTCAGAGGAGAATATCATCTGCGTTTTTTCATCTCTGCAGATCACTTGGTAA 3'

(2) NlaIII-NarI fragment of pSVeCPA-1

The smaller of the DNA fragments (approximately 0.33 kb) obtained by digestion with BglII and NarI of the TPA expression vector pSVeCPA-1 constructed as in item II-A above, is digested with NlaIII, which cleaves the base sequence of the cDNA encoding TPA in the vicinity of the 250th base pair, thus obtaining a DNA fragment of approximately 265 bp.

(3) BglII-NarI fragment of pSVeCPA-1

This is the larger of the DNA fragments (approximately 8.1 kb) obtained by digestion with BglII and NarI of the TPA expression vector pSVeCPA-1 constructed as shown in item II-A above.

Expression vectors pSVePN1, pSVePN2 and pSVePN3 for the three TPA derivatives PN1, PN2, and PN3, respectively, are constructed by ligation of the DNA fragments 1, 2, and 3. The expression vectors are introduced into E. coli DH1 (ATCC 33849), respectively, resulting in transformants.

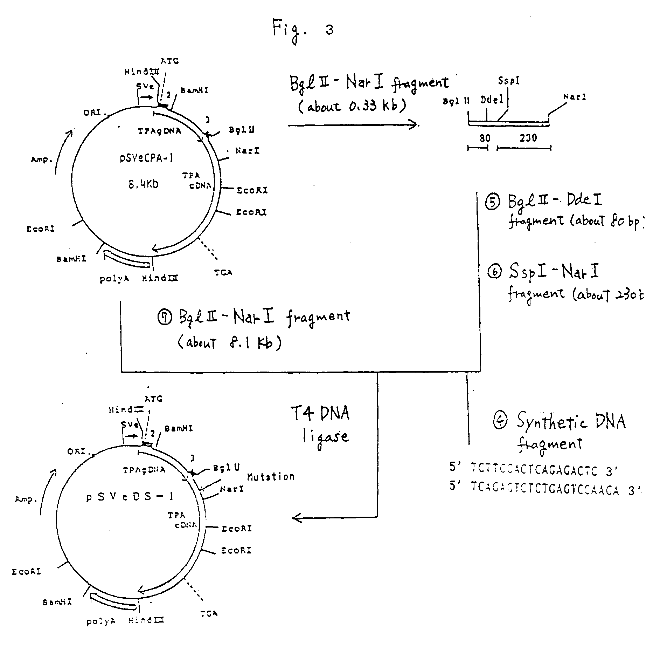

The expression vector pSVeDS1 used for the Production of the aforesaid TPA derivative DS1 can be constructed by ligating the following four types of DNA fragments 4, 5, 6, and 7, with the use of T4 DNA ligase. The construction of this expression vector is schematically shown in Figure 3.

(4) Synthetic DNA fragments

Two single-stranded synthetic DNA fragments with the following DNA sequences are synthesized. These two varieties of single-stranded DNA are complementary, and prior to the construction of the expression vector are annealed by a conventional method for use in the form of double-stranded DNA. Synthetic DNA fragment for construction of pSVeDS1:

5' TCTTGGACTCAGAGACTC 3'
5' TCAGAGTCTCTGAGTCCAAGA 3'

(5) BglII-DdeI fragment of pSVeCPA-1

The smaller of the DNA fragments (approximately 0.33 kb) obtained by digestion with BglII and NarI of the TPA expression vector pSVeCPA-1 constructed as in item II-A above, is digested with DdeI, which cleaves the cDNA sequence encoding TPA in the vicinity of the 270th base pair, thus obtaining a DNA fragment of approximately 80 bp.

(6) SspI-NarI fragment of pSVeCPA-1

The BglII-NarI fragment (0.33 kb) obtained as in item 5 above is digested with SspI, which cleaves the base sequence of the cDNA encoding TPA in the vicinity of the 290th base pair, thus obtaining a DNA fragment of approximately 230 bp.

(7) BglII-NarI fragment of pSVeCPA-1

This is the larger of the DNA fragments (approximately 8.1 kb) obtained by digestion with BglII and NarI of the TPA expression vector pSVeCPA-1 constructed as in item II-A above.

Expression vector pSVeDS1 for the TPA derivative DS1 is constructed by ligating the DNA fragments 4, 5, 6, and 7. The expression vector is introduced into E. coli DH1 (ATCC 33849), resulting in a transformant.

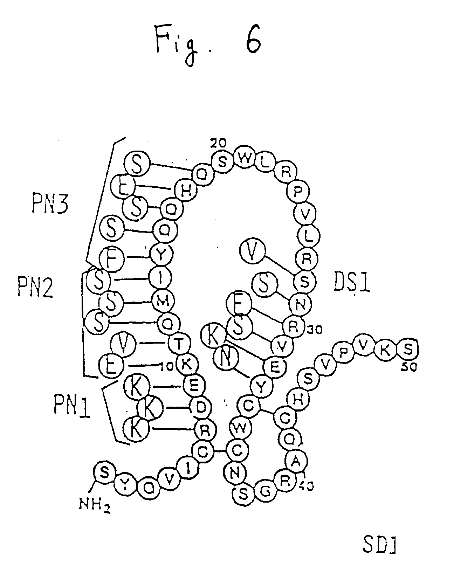

Figure 6 shows the amino acid sequences in the vicinity of the N-terminus of the TPA derivatives PN1, PN2, PN3 and DS1 produced by the aforesaid expression vectors, in comparison with that of native TPA.

Construction of expression vectors pSVeSD1-SD8

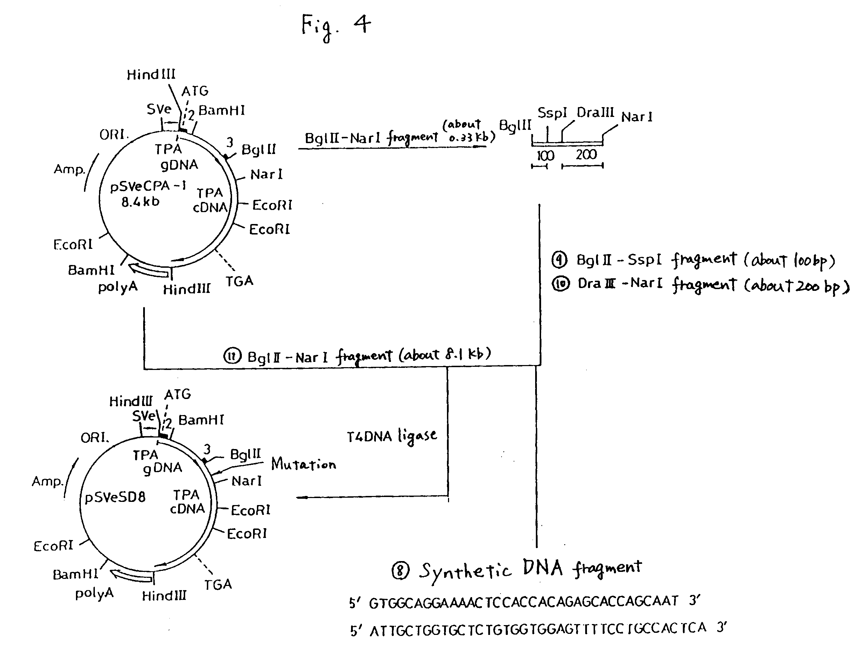

The expression vectors pSVeSD1, pSVeSD2, pSVeSD3, pSVeSD4, pSVeSD5, pSVeSD6, pSVeSD7, and pSVeSD8 used for the production of the aforesaid TPA derivatives SD1, SD2, SD3, SD4, SD5, SD6, SD7, and SD8, respectively, can be constructed by ligating the following four types of DNA fragments 8, 9, 10, and 11 by the use T4 DNA ligase. The construction of these expression vectors is schematically illustrated in Figure 4, employing pSVeSD8 as a typical example.

(8) Synthetic DNA fragments

Single-stranded synthetic DNA fragments with the following DNA sequences, two varieties for each vector, are synthesized. The two varieties of single-stranded DNA for each vector are complementary, and prior to the construction of the expression vector are annealed by a conventional method for use in the form of double-stranded DNA.

Synthetic DNA fragments for construction of pSVeSD1
5' GTGGCAGGAGGACTCCACCACAGAGCACCAGCAAT 3'
5, ATTGCTGGTGCTCTGTGGTGGAGTCCTCCTGCCACTCA 3'
Synthetic DNA fragments for construction of pSVeSD2
5' GTGGCACAGCACAATCACCACCAGGCACCAGCAGT 3'
5' ACTGCTGGTGCCTGGTGGTGATTGTGCTGTGCCACTCA 3'
Synthetic DNA fragments for construction of pSVeSD3
5′ GTGGCAGGAGGAGATCACCACAGAGCACCAGCAGT 3′
5′ ACTGCTGGTGCTCTGTGGTGATCTCCTCCTGCCACTCA 3′
Synthetic DNA fragments for construction of pSVeSD4
5′ GTGGCAGGAGACAATCACCACAGAGCACCAGCAGT 3′
5′ ACTGCTGGTGCTCTGTGGTGATTGTCTCCTGCCACTCA 3′
Synthetic DNA fragments for construction of pSVeSD5
5′ GTGGCACAGCACAATCACCACAGAGCACCAGCAGT 3′
5′ ACTGCTGGTGCTCTGTGGTGATTGTGCTGTGCCACTCA 3′
Synthetic DNA fragments for construction of pSVeSD6
5′ GTGGCAGAACAGCACAAACAGGAAGCACCAGCAGT 3′
5′ ACTGCTGGTGCTTCCTGTTTGTGCTGTTCTGCCACTCA 3′
Synthetic DNA fragments for construction of pSVeSD7
5′ GTGGCAGACAATGAACAGGAAGACACACCAGCAGT 3′
5′ ACTGCTGGTGTGTCTTCCTGTTCATTGTCTGCCACTCA 3′
Synthetic DNA fragments for construction of pSVeSD8
5′ GTGGCAGGAAAACTCCACCACAGAGCACCAGCAAT 3′
5′ ATTGCTGGTGCTCTGTGGTGGAGTTTTCCTGCCACTCA 3′

(9) BglII-SspI fragment of pSVeCPA-1

The smaller of the DNA fragments (approximately 0.33 kb) obtained by digestion with BglII and NarI of the TPA expression vector pSVeCPA-1 constructed as in item II-A above, is digested with SspI, which cleaves the cDNA sequence encoding TPA in the vicinity of the 290th base pair, thus obtaining a DNA fragment of approximately 100 bp.

(10) DraIII-NarI fragment of pSVeCPA-1

The BglII-NarI fragment (0.33 kb) obtained in item (9) above is digested with DraIII, which cleaves the bass sequence of the cDNA encoding TPA in the vicinity of the 320th base pair, thus obtaining a DNA fragment of approximately 200 bp.

(11) BglII-NarI fragment of pSVeCPA-1

This is the larger of the DNA fragments (approximately 8.1 kb) obtained by digestion with BglII and NarI of the TPA expression vector pSVeCPA-1 constructed in item II-A above.

Expression vectors pSVeSD1, pSVeSD2, pSVeSD3, pSVeSD4, pSVeSD5, pSVeSD6, pSVeSD7, and pSVeSD8 are constructed by ligating DNA fragments 8, 9, 10, and 11, respectively. The expression vectors are introduced into E. coli DH1 (ATCC 33849), respectively, resulting in transformants. A transformant carrying the expression vector pSVeSD4 was designated E. coli DH1 SD 459, and deposited with the Fermentation Research Institute, Agency of Industrial Science and Technology, 1-3, Higashi 1-chome, Tsukaba-shi, Ibaraki-ken, Japan, on January 31, 1990, under Accession No. BP-3082, where it will be maintained under the terms of the Budapest Treaty.

(II-C) Construction of expression vectors for TPA derivatives with modified kringle regions

Expression vectors for TPA derivatives formed by replacement of certain amino acid residues in the kringle 1 region of TPA by other amino acids are prepared, for example, by the method using synthetic DNA primers to introduce mutations into the DNA sequence encoding the amino acid sequence of TPA (i.e., site-directed mutagenesis).

For example, using this site-directed mutagenesis technique, the following two TPA derivatives KM4 and KM21 in which amino acid replacements have been introduced can be produced.

KM4: a TPA derivative in which glycine at the 161st position, lysine at the 162nd position, and serine at the 165th position from the N-terminus of the amino acid sequence of natural TPA are replaced by arginine, arginine, and tryptophan, respectively.

KM21: a TPA derivative in which asparagine at the 115th position from the N-terminus of the amino acid sequence of natural TPA is replaced by proline.

The aforesaid TPA derivatives with modified kringle regions can be prepared, for example, by excising a DNA fragment encoding a portion of the amino acid sequence of TPA (containing a site of the amino acid to be replaced) from a TPA expression vector, for example, pSVeCPA-1 obtained as in item II-A, and incorporating this fragment into a single-stranded phage or plasmid such as M13 or pUC119 suitable for introduction into an E. coli host. For example, the smaller of the fragments obtained by cleavage of pSVeCPA-1 with NarI and SmaI is inserted into M13mp11 (manufactured by Takara Shuzo, Co., Ltd.) which has likewise been treated with NarI and SmaI, thereby obtaining a recombinant phage vector M13NS. Next, a portion of a DNA fragment homologous to the aforesaid excised DNA fragment (containing a portion into which the mutations are to be introduced, and the bases present in the portion has been replaced by desired bases) is synthesized separately for use as a primer. For example, in order to obtain expression vector pSCKM4 for production of KM4, the following DNA fragment I is synthesized.

5′ TGCTGCAGAACTCCCAGCTGTACCTCCTCGCCTTAAAGACG 3′      (I)

Similarly, in order to obtain an expression vector pSCKM21 for production of KM21, the following DNA fragment II is synthesized.

5′ GCTGTTCCAGGGGGGTGCACTCG 3′      (II)

This primer, e.g., the aforesaid DNA fragment I, is phosphorylated with T4 polynucleotide kinase, then annealed to the aforesaid recombinant single-stranded phage or plasmid, and the DNA chain is extended by the conventional method. This procedure may be accomplished by the use of a commercially available in vitro mutagenetic system kit. The plasmid so obtained (M13-NSM4), containing the mutant portion, is then introduced into a suitable host, for example, E. coli JM109, and amplified. Next, this plasmid M13-NSM4 is treated with NarI and SmaI, the smaller fragment (approximately 1.2 kb; corresponding to the portion of the TPA-coding DNA containing the mutation) is isolated, and is ligated to the aforesaid NarI-SmaI fragment of pSVeCPA-1 (larger fragment, approximately 7 kb), thereby obtaining an expression vector (pSCKM4) for production of KM41. Similarly, using the DNA fragment II, an expression vector (pSCKM21) for producing KM21 is constructed.

Transformants are obtained by introducing each of the two expression vectors pSCKM4 and pSCKM21 constructed in this manner into E. coli DH1 (ATCC 33849).

(II-D) Construction of expression vectors for TPA derivatives with modifications of both finger region and kringle region

Expression vectors for finger-kringle 1 modified TPA derivatives, with amino acid replacements in both the finger region and the kringle 1 region, can be prepared by combining an expression vector for TPA with a modified finger region, prepared as in the above item II-B, and an expression vector for TPA with a modified kringle region, prepared as in the above item II-C, thereby constructing a new expression vector. For example, the four types of TPA derivatives KS48, KS218, KS44 and KS214, to be described below, can be produced by this method.

KS48: a TPA derivative in which asparagine at the 37th position, serine at the 38th position, glycine at the 39th position, arginine at the 40th position, alanine at the 41st position, glutamine at the 42nd position, glycine at the 161st position, lysine at the 162nd position, and serine at the 165th position from the N-terminus of the amino acid sequence of natural TPA are replaced by serine, valine, valine, glutamic acid, phenylalanine, serine, arginine, arginine, and tryptophan, respectively.

KS218: a TPA derivative in which asparagine at the 37th position, serine at the 38th position, glycine at the 39th position, arginine at the 40th position, alanine at the 41st position, glutamine at the 42nd position and asparagine at the 115th position from the N-terminus of the amino acid sequence of natural TPA are replaced by serine, valine, valine, glutamic acid, phenylalanine, serine, and proline, respectively.

KS44: a TPA derivative in which asparagine at the 37th position, serine at the 38th position, glycine at the 39th position, arginine at the 40th position, alanine at the 41st position, glutamine at the 42nd position, glycine at the 161st position, lysine at the 162nd position, and serine at the 165th position from the N-terminus of the amino acid sequence of natural TPA are replaced by serine, valine, valine, isoleucine, valine, serine, arginine, arginine, and tryptophan, respectively.

KS214: a TPA derivative in which asparagine at the 37th position, serine at the 38th position, glycine at the 39th position, arginine at the 40th position, alanine at the 41st position, glutamine at the 42nd position and asparagine at the 115th position from the N-terminus of the amino acid sequence of natural TPA are replaced by serine, valine, valine, isoleucine, valine, serine, and proline, respectively.

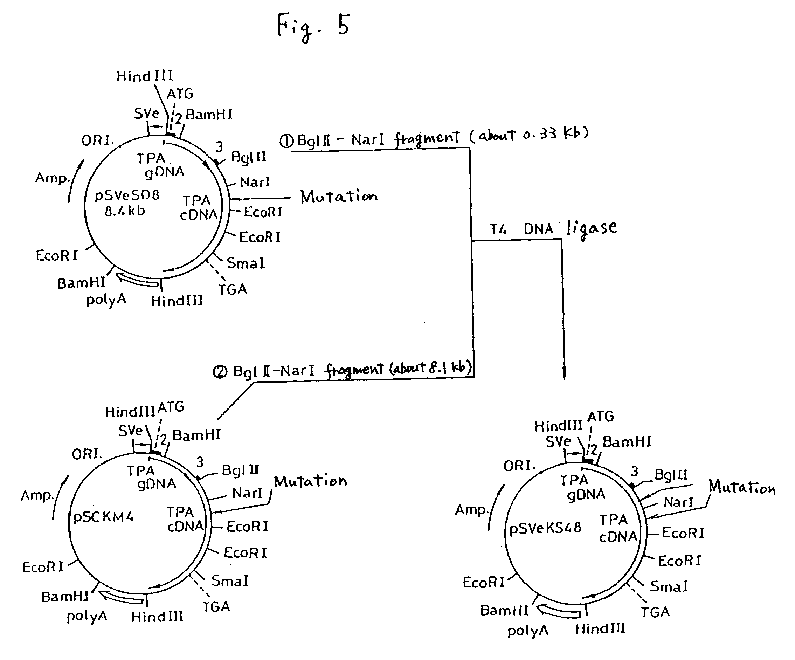

Expression vectors pSVeKS48, pSVeKS218, pSVeKS44 and pSVeKS214 for the production of these TPA derivatives can be constructed by ligating the two types of fragments 1 and 2 indicated below. The construction of these expression vectors is schematically illustrated in Figure 5, employing pSVeKS48 as a typical example.

(1) A smaller fragment (approximately 0.33 kb) obtained by digestion of the expression vector pSVeSD8 or pSVeSD4 for the finger region modified TPA with BglII and NarI,

(2) A larger fragment (approximately 8.1 kb) obtained by digestion of the expression vector pSCKM4 or pSCKM21 for kringle region modified TPA with BglII and NarI.

Four TPA derivative expression vectors pSVeKS48, pSVeKS218, pSVeKS44 and pSVeKS214 are obtained by ligating the DNA fragments of the above two varieties 1 and 2, respectively. The expression vectors are introduced into E. coli DH1 (ATCC 33849) respectively, resulting in transforments.

A transformant carrying the expression vector pSVeKS48 was designated E. coli DH115 KS48, and deposited with the Fermentation Research Institute, Agency of Industrial Science and Technology, 1-3, Higashi 1-chome, Tsukuba-shi, Ibaraki-ken, Japan, on June 25, 1990, under Accession No. BP-3083, where it will be maintained under the terms of the Budapest Treaty.

(III) Marker genes

Genes such as Ecogpt [Mulligaan, R. C., et al., Science, 209, 1422 (1980)], neo [Southern, P. J., et al., Journal of Molecular and Applied Genetics 1, 327 (1982)], and dhfr [Wigler, M., et al., Proc. Natl. Acad. Sci. USA, 77, 327 (1980)] can be used as markers for the selection of transformants obtained by the introduction of the aforesaid expression vectors into animal cells. These marker genes can be included in either the expression vectors for TPA derivatives or in plasmids other than the said vectors. In the latter case, a plasmid vector possessing a selectable marker gene is constructed and used after mixing with the TPA expression vector in an appropriate proportion, thus permitting selection of the transformants. For example, a marker vector pSV2neo-dhfr possessing the neo and dhfr genes is suitable for this purpose, and can be constructed in the following manner. The rDNA vector pSV2dhfr (American Type Culture Collection, rDNA Vector 37146), possessing the dhfr gene, is cleaved with PvuII. The resulting PvuII-PvuII fragments are ligated BamHI linker-d (pCGGATCCG) using T4 DNA ligase, thereby obtaining a vector pSV2Bdhfr. This vector is cleaved with BamHI, and the resulting BamHI-BamHI fragment is ligated, using T4 DNA ligase, with a BamHI-BamHI fragment obtained by cleaving the rDNA vector pSV2neo (American Type Culture Collection, rDNA Vector 37149) with <u>Bam</u>HI, thereby obtaining a circular marker vector pSV2neo-dhfr.

(IV) Transformation of cultured animal cells and production of TPA derivatives

Cultured animal cells are used as hosts, and CH0-K1 (ATCC CCL-61) is particularly suitable. By the use of this type of cultured animal cells, the TPA derivatives can be produced in glycosylated form.

Although the various methods used for the introduction of the aforesaid expression vectors into animal cells differ with respect to transfection efficiency, those applicable for the present purpose include the calcium phosphate method [Wigler, M., et al., Cell, <u>11</u>, 233 (1977)], the microinjection method [Anderson, W. F., at al., Proc. Natl. Acad. Sci. USA, <u>77</u>, 5399 (1989)], the liposome method, the DEAE-dextran method, the cell fusion method [Schoffner, W., et al., Proc. Natl. Acad. Sci. USA, <u>77</u>, 2163 (1980)], and the electroporation method [Tatsuya, M., et al., Cell Technology, <u>6</u>, 494 (1987)]. After the TPA expression vector has been introduced into the host cells by one of these methods, the transformants are selected according to the characters conferred by the marker gene mentioned in above item III.

By the measurement of the plasminogen-activating activity (i.e., the degree of activity of the plasminogen-activating effect) in the culture medium of the respective transformant cells, it can be determined if the transformants so obtained actually produce the desired TPA derivatives. Techniques available for the measurement of plasminogen-activating activity include the utilization of fibrin plates containing plasminogen (Mackie, M., et al., British Journal of Hematorogy, <u>47</u>, 77 (1981)), the measurement of decomposition of the synthetic substrate S-2251 on which plasmin acts [Allen, R. A., and Pepper, D. S., Thrombosis and Haemostasis, <u>45</u>, 43 (1981)], the CLT method [Gaffney, P. T., and Cartis, A. D., Thrombosis and Haemostasis <u>53</u>, 134 (1985)], and ELISA [Holvoest, T., et al., Thrombosis and Haemostasis, <u>54</u>, 684 (1985)].

(V) <u>Recovery and purification of TPA derivatives</u>

Strains producing the TPA derivatives can be cultivated by any method appropriate for the animal cells used as hosts. Recovery and purification of the TPA derivatives from the culture can be accomplished by a suitable combination of techniques such as column chromatography using an appropriate support (e.g., CPG-10, chelating Sepharose, Con-A Sepharose, ion-exchangers, Octyl Sepharose, Sephadex gel, etc.), antibody column chromatography, electrophoresis, etc. In experiments according to the present invention, 1-3 mg of purified TPA derivative was obtained from 2-6 liters of culture broth for each of strains producing the respective TPA derivatives.

(VI) <u>Evaluation of the TPA derivative</u>

Various properties relating to dissolution of thrombi include specific activity, affinity for fibrin, dependence of activity on fibrin, resistance to protease, persistence in the blood, plasminogen-activating activity, in vitro thrombolytic ability, sensitivity to inhibitors, etc. Among these, the affinity for fibrin can be assessed by the method of Collen, D. et al. Blood, <u>71</u>, 216 (1988), using uptake by a fibrin clot as an index. In vitro fibrolytic ability can be measured by the method of Larsen, G. R. et al. [J. Biol. Chem., <u>263</u>, 1023 (1988)], using release of radioactivity from $^{125}$I-fibrin; or by the method of Collen, D. et al. [Thromb. Haemost., <u>52</u>, 308 (1984)]. Fibrin dependence of TPA activity or plasminogen-activating activity can be assessed by the method of Collen, D. et al., using the synthetic substrate S-2251 on which plasmin acts [J. Biol. Chem., <u>257</u>, 2912 (1982), sapra] or by the method of Takada, A. et al. [Haemostasis, <u>18</u>, 117 (1988)]. Persistence in the blood can be evaluated as half-life using the method described by Beebe, D. P. et al. [Thrombosis Research, <u>43</u>, 663 (1986)] or by Mattson, Ch. et al. [Thrombosis Research, <u>30</u>, 91 (1983)].

Various properties relating to dissolution of thrombi include specific activity, affinity for fibrin, dependence of activity on fibrin, resistance to protease, persistence in the blood, plasminogen-activating activity, in vitro thrombolytic activity, sensitivity to inhibitors, etc. The TPA derivatives of the present invention possess thrombolytic characteristics of nearly the same level as those of native TPA, and moreover, display excellent persistence in the blood. These characteristics will be further clarified in the following examples.

<u>EXAMPLES</u>

The present invention will now be explained in further detail with reference to specific examples.

All experiments in connection with the present invention were performed in accordance with the "Guidelines for Recombinant DNA Experiments" stipulated under the authority of the Prime Minister of Japan. Also, the following learned journals and treatises were used for reference with regard to the detailed procedures adopted in the management and use of phages, plasmids, DNA, various enzymes, E. coli and other materials employed in the examples and comparative examples.

1. Protein, nucleic acid, and enzyme, 26 (4), (1981), special issue, Genetic Engineering, Kyoritsu Shuppan (Japan).

2. Experimental Method for Genetic Engineering, written and ed. by Takagi, Y. (1980), Kodansha (Japan).

3. Manual for Genetic engineering, written and ed. by Takagi, Y. (1982), Kodansha (Japan).

4. Molecular cloning, a Laboratory Manual, T. Maniatis et al., ed., (1982), Cold Spring Harbor Laboratory.

5. Methods in Enzymology, L. Grossmam et al., ed., 65 (1980), Academic Press.

6. Methods in Enzymology, R. Wu, ed., 65 (1979, Academic Press.

Example 1

(I) Preparation of DNA fragment encoding TPA

A DNA fragment encoding native TPA was prepared from cDNA in the following manner.

First, using the guanidine-hot phenol method, total RNA was extracted from CHO-K1 cells which had been transformed with a TPA expression vector pSVePA-1, which utilizes genomic DNA (gDNA) (Japanese Laid-Open Patent Publication No. 62-14783, supra). This total RNA extract was subjected to oligo-dT cellulose chromatography, and the polyA + mRNA fraction so obtained was subjected to molecular weight fractionation by the sucrose gradient centrifugation method, thereby obtaining a fraction containing mRNA of TPA. Using this mRNA fraction, cDNA was prepared with a commercial cDNA synthesis kit Amersham, and this was used for preparing a cDNA library. A commercial cDNA cloning kit (Amersham) employing lambda-gt10 was used for preparing this cDNA library. Next, this library was subjected to plaque hybridization by the conventional method. Using, as a probe, an approximately 2.5 kb DNA fragment containing the 10th, 11th and 12th exons, obtained by cleavage of the aforesaid expression vector pSVePA-1 with an restriction enzyme XbaI, the phage clones which displayed positive hybridization with the said DNA fragment were selected.

DNA was isolated from each of the several positive phage clones obtained in this manner, and after digestion with a restriction enzyme HindIII (Takara Shuzo, Co., Ltd.), the DNA was subjected to agarose gel electrophoresis. Then, the positive phage clones were analyzed by Southern hybridization, using as a probe, the same XbaI-cleaved 2.5 kb DNA fragment which was employed in the aforesaid plaque hybridization procedure. The results showed that a clone designated as CH79 hybridized with the aforesaid probe, and contained a DNA fragment which was cleaved into an approximately 2.2 kb fragment by HindIII. This approximately 2.2 kb HindIII-cleaved DNA fragment was isolated by agarose gel electrophoresis, and using T4 DNA ligase, ligated to a plasmid vector pUC19 (Takara Shuzo, Co., Ltd.) which had likewise been digested with HindIII. The ligation product was then introduced into E. coli DH1, thus obtaining a cDNA clone pCH79. The base sequence of the cDNA portion of this clone was determined with a commercial sequencing kit (Takara Shuzo, Co., Ltd.) employing the M13 method. The results revealed that the 5′ terminus of this cDNA portion was linked with a HindIII recognition site originating from the plasmid vector pUC19, while approximately 150 bp downstream from this portion, a BglII recognition site was present, and approximately 1500 bp further downstream the termination codon TGA was present. Approximately 410 bp downstream from this TGA codon, another HindIII recognition site derived from the plasmid vector pUC19 was present. The base sequence of this cDNA portion was identical with that reported by Pennica et al. (Nature, 301, 214, supra), except that the cytosine in the 585th position was replaced by thymine and the adenine in the 1725th position by cytosine.

(II) Construction of expression vectors

A TPA expression vector pSVeCPA-1 and expression vectors for TPA derivatives were constructed as follows.

(II-A) Construction of TPA expression vector pSVeCPA-1

A TPA expression vector pSVeCPA-1 was constructed by the ligation of three DNA fragments obtained in the following items 1, 2, and 3. The construction of this expression vector is schematically shown in Figure 1.

13

(1) HindIII-HindIII fragment derived from pSVeSalI

A plasmid vector pSVeSalI (Japanese Laid-Open Patent Publication No.62-14783, supra) was cleaved with a restriction enzyme NcoI, and the larger DNA fragment so obtained (NcoI-NcoI fragment, approximately 4.7 kb) was isolated. This DNA fragment contained an early promoter region (SVe) including an origin (Ori) for replication in E. coli, derived from SV40; a sequence containing a polyadenylation signal (polyA); an ampicillin-resistance gene (Amp.); and two HindIII recognition sites. This NcoI-NcoI fragment was circularized with T4 DNA ligase, and the plasmid vector so obtained, designated as pSVeSal-IHindIII, was amplified by introduction into E. coli DH1. Next, this plasmid vector pSVeSalI-HindIII was isolated from the E. coli DH1 by the conventional method, cleaved with HindIII, and the larger of the resulting fragments (HindIII-HindIII fragment, approximately 4.7 kb) was recovered.

(2) HindIII-BglII fragment of pSVePA-1

The aforesaid TPA expression vector pSVePA-1, constructed by utilizing gDNA, was cleaved with HindIII and BglII, thereby obtaining a small (approximately 1.9 kb) DNA fragment (HindIII-BglII fragment). This HindIII-BglII fragment contained the entire second exon and a portion of the third exon of gDNA encoding TPA.

(3) BglII-HindIII fragment of pCH79

First, pCH79, the cDNA clone obtained as in the above item I, was cleaved with BglII and HindIII, thereby obtaining a DNA fragment (BglII-HindIII fragment) of approximately 2 kb containing TPA cDNA.

A TPA expression vector, designated as pSVeC-PA-1, was then constructed by ligating the aforesaid DNA fragments 1, 2, and 3 with T4 DNA ligase. This vector pSVeCPA-1 was introduced into an E. coli DH1, and used in the construction of the following expression vectors for TPA derivatives. This TPA expression vector pSVeCPA-1 comprises a gene having the ability of producing native TPA, and contains a portion derived from gDNA (from the second exon indicated by numeral 2 to the BglII recognition site of the third exon indicated by numeral 3 in Figure 1) and a portion derived from cDNA.

(II-B) Construction of expression vectors for finger region modified TPA derivatives

The twelve TPA derivatives composed of the amino acid sequence of native TPA with the respective amino acid replacements indicated in Table 1, 2, or 3 were designated by PN1, PN2, PN3, DS1, SD1, SD2, SD3, SD4, SD5, SD6, SD7 and SD8.

Table 1

| TPA derivative | Expression Vector | Amino acid number from N-terminus | | | | | | | | | | | | |
|---|---|---|---|---|---|---|---|---|---|---|---|---|---|---|
| | | 7 | 8 | 9 | 10 | 11 | 12 | 13 | 14 | 15 | 16 | 17 | 18 | 19 |
| Native TPA | pSVeCPA-1 | Arg | Asp | Glu | Lys | Tyr | Gln | Met | Ile | Tyr | Gln | Gln | His | Gln |
| PN1 | pSVePN1 | Lys | Lys | Lys | — | — | — | — | — | — | — | — | — | — |
| PN2 | pSVePN2 | — | — | — | Glu | Val | Ser | Ser | Ser | — | — | — | — | — |
| PN3 | pSVePN3 | — | — | — | — | — | — | — | — | Phe | Ser | Ser | Glu | Ser |

— denote the same amino acid as that of native TPA.

Table 2

| TPA derivative | Expression Vector | Amino acid number from N-terminus | | | | | |
|---|---|---|---|---|---|---|---|
| | | 28 | 29 | 30 | 31 | 32 | 33 |
| Native TPA | pSVeCPA-1 | Ser | Asn | Arg | Val | Glu | Tyr |
| DS1 | pSVeDS1 | Val | Ser | Glu | Ser | Lys | Asn |

Table 3

| TPA derivative | Expression Vector | Amino acid number from N-terminus | | | | | | | | | |
|---|---|---|---|---|---|---|---|---|---|---|---|
| | | 35 | 36 | 37 | 38 | 39 | 40 | 41 | 42 | 43 | 44 |
| Native TPA | pSVeCPA1 | Trp | Cys | Asn | Ser | Gly | Arg | Ala | Gln | Cys | His |
| SD1 | pSVeSD1 | Trp | Cys | Ser | Val | Val | Glu | Ser | Ser | Cys | His |
| SD2 | pSVeSD2 | Trp | Cys | Leu | Val | Val | Ile | Val | Leu | Cys | His |
| SD3 | pSVeSD3 | Trp | Cys | Ser | Val | Val | Ile | Ser | Ser | Cys | His |
| SD4 | pSVeSD4 | Trp | Cys | Ser | Val | Val | Ile | Val | Ser | Cys | His |
| SD5 | pSVeSD5 | Trp | Cys | Ser | Val | Val | Ile | Val | Leu | Cys | His |
| SD6 | pSVeSD6 | Trp | Cys | Phe | Leu | Phe | Val | Leu | Phe | Cys | His |
| SD7 | pSVeSD7 | Trp | Cys | Val | Phe | Leu | Phe | Ile | Val | Cys | His |
| SD8 | pSVeSD8 | Trp | Cys | Ser | Val | Val | Glu | Phe | Ser | Cys | His |

The corresponding expression vectors pSVePNI, pSVePN2, pSVePN3, pSVeDS1, pSVeSD1, pSVeSD2, pSVeSD3, pSVeSD4, pSVeSD5, pSVeSD6, pSVeSD7 and pSVeSD8, used for the production of these TPA derivatives, were constructed as follows.

Among the aforesaid expression vectors, pSVePN1, pSVePN2 and pSVePN3 were constructed by using T4 DNA ligase to ligate the three types of DNA fragments obtained in the following items 1, 2, and 3.

17

(1) Synthetic DNA fragments

Single-stranded synthetic DNA fragments for use in the construction of the expression vectors for the respective TPA derivatives, two varieties for each vector, were synthesized with a commercial DNA synthesizer (381A DNA Synthesizer, Applied Biosystems). The DNA sequences of these synthetic oligonucleotides are shown below. The two varieties of single-stranded DNA corresponding to each vector are complementary. Prior to the construction of the expression vector, these DNA fragments were annealed by a conventional method for use in the form of double-stranded DNA.

Synthetic DNA fragments for construction of pSVePN1

```
5' GATCTTACCAAGTGATCTGCAAGAAGAAGAAAACGCAGATGATATACCAGCAACATCAGTCATG 3'
5' ACTGATGTTGCTGGTATATCATCTGCGTTTTCTTCTTCTTGCAGATCACTTGGTAA 3'
```

Synthetic DNA fragments for construction of pSVePN2

```
5' GATCTTACCAAGTGATCTGCAGAGATGAAGAGGTCTCCTCCTCCTACCAGCAACATCAGTCATG 3'
5' ACTGATGTTGCTGGTAGGAGGAGGAGACCTCTTCATCTCTGCAGATCACTTGGTAA 3'
```

Synthetic DNA fragments for construction of pSVePN3

```
5' GATCTTACCAAGTGATCTGCAGAGATGAAAAAACGCAGATGATATTCTCCTCTGAGTCCTCATG 3'
5' AGGACTCAGAGGAGAATATCATCTGCGTTTTTTCATCTCTGCAGATCACTTGGTAA 3'
```

(2) NlaIII-NarI fragment of pSVeCPA-1

The smaller of the DNA fragments (BglII-NarI fragment, approximately 0.33 kb) obtained by digestion with BglII and NarI of the TPA expression vector pSVeCPA-1, constructed as in item II-A above, was digested with NlaIII, which cleaves the base sequence of the cDNA encoding TPA in the vicinity of the 250th base pair, thus obtaining an NlaIII-NarI fragment of approximately 265 bp.

(3) BglII-NarI fragment of pSVeCPA-1

The larger of the DNA fragments (BglII-NarI fragment, approximately 8.1 kb) was obtained by digestion with BglII and NarI of the TPA expression vector pSVeCPA-1 constructed as in item II-A above.

Three expression vectors (pSVePN1, pSVePN2 and pSVePN3) for producing the corresponding TPA derivatives were constructed by ligation of the DNA fragments 1, 2, and 3 obtained in this manner. Transformants were then obtained by introducing these expression vectors into E. coli DH1 (ATCC 33849).

The expression vector pSVeDS1 (production for DS1) was constructed by using T4 DNA ligase to ligate the following four types of DNA fragments 4, 5, 6, and 7.

(4) Synthetic DNA fragments

Single-stranded synthetic DNA fragments for use in the construction of the expression vectors for the respective TPA derivatives, two varieties for each vector, were synthesized with a commercial DNA synthesizer (381A DNA Synthesizer, Applied Biosystems). The sequences of these synthetic oligonucleotides are shown below. The two varieties of single-stranded DNA corresponding to each vector are complementary, and prior to the construction of the expression vector were annealed by a conventional method for use in the form of double-stranded DNA.

Synthetic DNA fragments for construction of DS1:
5' TCTTGGACTCAGAGACTC 3'
5' TCAGAGTCTCTGAGTCCAAGA 3'

(5) BglII-DdeI fragment of pSVeCPA-1

The smaller of the DNA fragments (BgIII-NarI fragment, approximately 0.33 kb) obtained by digestion with BgIII and NarI of the TPA expression vector pSVeCPA-1, constructed as in item II-A above. This BgIII-NaII fragment was digested with DdeI, which cleaves the cDNA sequence encoding TPA in the vicinity of the 270th base pair, thus obtaining a BgIII-DdeI fragment of approximately 80 bp.

(6) SspI-NarI fragment of pSVeCPA-1

The BgIII-NarI fragment (0.33 kb) obtained as in item II-B (5) above was digested with SspI, which cleaves the base sequence of the cDNA encoding TPA in the vicinity of the 290th base pair, thus obtaining an SspI-NarI fragment of approximately 230 bp.

(7) BgIII-NarI fragment of pSVeCPA-1

The larger of the DNA fragments (approximately 8.1 kb) was obtained by digestion with BgIII and NarI of the TPA expression vector pSVeCPA-1, constructed as in item II-A above.

The expression vector pSVeDS1 was constructed by ligation of the DNA fragments 4, 5, 6, and 7 obtained in this manner. A transformant was obtained by introduction of this TPA derivative expression vector into an E. coli DH1 (ATCC 33849).

The aforesaid expression vectors pSVeSD1, pSVeSD2, pSVeSD3, pSVeSD4, pSVeSD5, pSVeSD6, pSVeSD7, and pSVeSD8 were constructed by using T4 DNA ligase to ligate the following four types of DNA fragments 8, 9, 10, and 11.

The sequences of these synthetic oligonucleotides and the construction of the restriction fragments used for the preparation of the expression vectors pSVeSD1, pSVeSD2, pSVeSD3, pSVeSD4, pSVeSD5, pSVeSD6, pSVeSD7 and pSVeSD8 are shown above under the heading "Construction of expression vectors pSVeSD1-SD8", items (8), (9), (10) and (11).

(II-C) Construction of expression vectors for TPA derivatives with modified kringle 1 region

Expression vectors pSCKM4 and pSCKM21 for the TPA kringle 1 regions, were constructed as follows. Amino acid replacements are shown in Table 4.

Table 4

| TPA derivative | Expression Vector | Amino acid number from N-terminus | | | |
|---|---|---|---|---|---|
| | | 115 | 161 | 162 | 165 |
| Native TPA | pSVeCPA-1 | Asn | Gly | Lys | Ser |
| KM4 | pSCKM4 | — | Arg | Arg | Trp |
| KM21 | pSCKM21 | Pro | — | — | — |

— denote the same amino acid as that of native TPA.

(1) Preparation of vector M13NS for introduction of mutations

The TPA expression vector pSVeCPA-1 was cleaved with restriction enzymes NarI (New England Biolabs) and SmaI (Takara Shuzo, Co., Ltd.), and a fragment of approximately 1.2 kb was isolated by agarose gel electrophoresis. This fragment contains a cDNA sequence encoding an amino acid sequence of native TPA from the glycine residue at the 110th position to the proline residue at the 508th position. Using T4 DNA ligase, this approximately 1.2 kb fragment was ligated to M13mp11 (Takara Shuzo, Co., Ltd.) which had been cleaved with NarI and SmaI, thereby obtaining a vector M13-NS. This vector was introduced into E. coli JM109 (Takara Shuzo, Co., Ltd.), resulting in a transformant.

20

(2) Site-directed amino acid replacements

The transformants possessing the vector M13-NS obtained as in item II-C, 1 were cultivated, and single-stranded DNA was obtained from the supernate of the culture medium. Next, in order to construct the expression vector pSCKM4 for KM4, the following DNA fragment I was synthesized with a commercial DNA synthesizer (381A DNA Synthesizer, Applied Biosystems).

5′ TGCTGCAGAACTCCCAGCTGTACCTCCTCGCCTTAAAGACG 3′     (I)

This DNA fragment was phosphorylated with T4 polynucleotide kinase (Takara Shuzo, Co., Ltd.), then annealed to the aforesaid single-stranded DNA M13-NS, and the DNA chain was extended. This procedure was accomplished by means of a commercial in vitro mutagenetic system kit (Amersham). The plasmid (M13-NSM4) containing the desired mutant portion, obtained in this manner was introduced into E. coli TG1 and amplified. Thus, the DNA sequence of the original expression vector corresponding to the amino acid sequence from the 161st through the 165th residue of native TPA (glycine, lysine, tyrosine, serine, serine), i.e., -GGGAAGTACAGCTCA-, was converted into -AGGAGGTACAGCTGG-. This DNA sequence corresponds to the amino acid sequence arginine, arginine, tyrosine, serine, tryptophan. The introduction of the aforesaid mutation in the DNA sequence was confirmed by means of a commercial sequencing kit (Takara Shuzo, Co., Ltd.).

Next, in order to construct an expression vector pSCKM21 for the TPA derivative KM21, the following DNA fragment (II) was synthesized with a commercial DNA synthesizer (381A DNA Synthesizer, Applied Biosystems).

5′ GCTGTTCCAGGGGGGTGCACTCG 3′     (II)

Using this DNA fragment II, the mutant vector M13-NSM21 was prepared by the same procedure as that described above.

(3) Preparation of expression vectors for TPA derivatives with modified kringle regions

After preparation of the double-stranded form of the plasmid M13-NSM4 obtained as in the aforesaid item II-C, 2, this DNA was cleaved with NarI and SmaI, and a fragment of approximately 1.2 kb (smaller fragment) was obtained by agarose gel electrophoresis. Next, the TPA expression vector pSVeCPA-1 was also treated with NarI and SmaI, and a fragment of approximately 7 kb (larger fragment) was obtained by agarose gel electrophoresis. An expression vector pSCKM4 for KM4 was then obtained by ligation of these two fragments. Similarly, using M13-NSM21 in place of M13-NSM4, an expression vector pSCKM21 was obtained and introduced into E. coli DH1. This vector comprises a gene encoding the TPA derivative KM21. Transformants were obtained by introduction of pSCK4 and PSCKM21 into E. coli DH1 (ATCC 33849), respectively.

(IID) Construction of expression vectors for TPA derivatives with modifications in both finger and kringle regions

Expression vectors pSVeKS48, pSVeKS218, pSVeKS44, and pSVeKS214 for the TPA derivatives KS48, KS218, KS44, and KS214, respectively, with amino acid replacements in both the finger region and the kringle 1 region, were constructed as follows. Amino acid replacements in these derivatives are shown in Table 5.

Table 5

| TPA derivative | Expression Vector | Amino acid number from N-terminus | | | | | | | | | |
|---|---|---|---|---|---|---|---|---|---|---|---|
| | | 37 | 38 | 39 | 40 | 41 | 42 | 115 | 161 | 162 | 165 |
| Native TPA | pSVeCPA1 | Asn | Ser | Gly | Arg | Ala | Gln | Asn | Gly | Lys | Ser |
| KS48 | pSVeKS48 | Ser | Val | Val | Glu | Phe | Ser | Asn | Arg | Arg | Trp |
| KS218 | pSVeKS218 | Ser | Val | Val | Glu | Phe | Ser | Pro | Gly | Lys | Ser |
| KS44 | pSVeKS44 | Ser | Val | Val | Ile | Val | Ser | Asn | Arg | Arg | Trp |
| KS214 | pSVeKS214 | Ser | Val | Val | Ile | Val | Ser | Pro | Gly | Lys | Ser |

pSVeSD8 was treated with BgIII and NarI, and the smaller fragment (approximately 0.33 kb) was isolated. Similarly, pSCK4 was treated with BgIII and NarI, and the larger fragment (approximately 8.1 kb) was isolated. These two fragments were ligated with T4 DNA ligase, thereby obtaining an expression vector pSVeKS48, and transformants were obtained by introducing this vector into E. coli DH1 (ATCC No. 33849). A transformant carrying the expression vector pSVeKS48 was designated E. coli DH1 KS48, and deposited with the Fermentation Research Institute, Agency of Industrial Science and Technology, 1-3, Higashi 1-chome, Tsukubashi, Ibaraki-ken, Japan, on June 25, 1990, under Accession No. BP-3083, where it will be maintained under the terms of the Budapest Treaty.

Separately, the smaller fragment (approximately 0.33 kb) obtained by treatment of pSVeSD8 with BgIII and NarI was isolated, while pSCKM21 was also treated with BgIII and NarI and the larger of the resulting fragments (approximately 8.1 kb) was isolated. An expression vector pSVeKS218 was obtained by ligation of these two fragments with T4 DNA ligase. Transformants were obtained by introducing this vector into E. coli DH1 (ATCC No. 33849).

Using the same method, the expression vector pSVeKS44 was constructed from pSVeSD4 and pSCKM4, and the expression vector pSVeKS214 was constructed from pSVeSD4 and pSCKM21.

(III) Construction of marker vector pSV2neo-dhfr

Marker vectors possessing the neo and dhfr genes as selectable markers were constructed in the following manner.

First, the rDNA vector pSV2dhfr (ATCC, rDNA Vector 37146) was cleaved with a restriction enzyme PvuII, and a PvuII-PvuII fragment was isolated. Using T4 DNA ligase, this PvuII-PvuII fragment was ligated with BamHI linker-d (pCGGATCCG, Takara Shuzo, Co., Ltd.), thereby constructing a vector pSV2Bdhfr, which was introduced into E. coli DH1 and amplified. This vector pSV2Bdhfr was isolated and digested with BamHI, and a BamHI-BamHI fragment (approximately 2 kb) containing dhfr gene was then isolated by agarose gel electrophoresis. Using T4 DNA ligase, this BamHI-BamHI fragment was ligated to a BamHI-BamHI fragment obtained by cleavage of the rRNA vector pSV2neo (American Type Culture Collection, rDNA Vector 37149). The circular marker vector constructed in this manner was introduced into E. coli DH1. In this marker vector, which was designated pSV2neo-dhfr, the neo and dhfr genes are inserted in the same expression direction.

(IV) Transformation of cultured animal cells and production of TPA derivatives

Cultured animal cells of the line CHO-K1 (ATCC CCL-61) were selected as the host cells, and transformed using the various TPA derivative expression vectors obtained as in the aforesaid item II-B, in accordance with the method of Chen, C. et al. [Molecular and Cellular Biology, 7, 2745 (1987)]. Each of these TPA derivative expression vectors was used in a mixture with the marker vector pSV2neo-dhfr in a ratio of 300:1 [TPA derivative expression vector:pSV2neo-dhfr(by weight)]. A coprecipitate of the vector mixture and calcium phosphate was then added to CHO-K1 cells ($5 \times 10^5$ cells/10 ml medium/10 cm diameter petri dish) previously grown in MD medium (MCDB302:modified Dulbecco's MEM = 1:1, Sigma) with 5% of added fetal calf serum (FCS), and after 15 hours of cultivation, the medium was replaced with fresh medium of the same type. After a further 48 hours of cultivation, the medium was replaced by MD medium containing 5% FCS, 800 $\mu$g/ml G418 sulfate (Gibco), 7 mM $\epsilon$-aminocaproic acid, and 50 $\mu$M foipan (Ono Pharmaceutical Co.). Then, cultivation was continued for a further 2 weeks, after which the G418-resistant strains were isolated. These G418-resistant strains were transferred to 12-well multidishes (Linbrow Co.), and cultivated over the entire base area of the dishes for 24 hours in the aforesaid MD medium. The content of TPA derivative in this culture broth was quantitated by activity measurements, using fibrin plates containing plasminogen [Mackie, M., et al., British Journal of Hematology, 47, 77 (1981)]. Protein-assayed preparations of TPA derivatives, purified as described in item V below, were used for this quantitation. With respect to each transformant, cultures producing TPA in a concentrations of at least 0.5 $\mu$g/ml per milliliter culture medium were selected. The transformant cells thus selected produced TPA derivatives even in serum-free MD medium (MD medium containing 7 mM $\epsilon$-aminocaproic acid, 50 $\mu$M foipan, 1 mg/ml bovine serum albumin, and 5 $\mu$g/ml insulin).

(V) Recovery and purification of TPA derivatives

Each of the TPA derivatives produced by the aforesaid transformants was recovered and purified by the following procedure. A commercial ELISA kit (Imubind TPA ELISA Kit, American Diagnostic Inc.) was used for the detection of TPA antigens in this process.

The culture broth containing the TPA derivative, obtained by cultivation of the transformants in the MD serum-free medium described in item IV above, was passed through a CPG-10 column (Electronucleonics Co.) equilibrated with 20 mM phosphate buffer solution (PH 7.5) containing 1 M sodium chloride and 50 $\mu$M foipan, and the column was washed with the same buffer solution as was used for equilibration. Almost no TPA derivative was detected by measurement of TPA antigen in the culture broth which had passed through the column and in the washing solution. Next, using a 20 mM phosphate buffer solution (pH 7.5) containing 1 M sodium chloride, 0.5 M potassium thiocyanate, 1 M $\epsilon$-aminocaproic acid and 50 $\mu$M foipan as eluent, the TPA derivative was eluted from the CPG-10 column. This eluate solution was then charged into a ConA-Sepharose (Pharmacia) column equilibrated with 20 mM phosphate buffer solution (pH 7.5) containing 1 M sodium chloride, 0.01% Tween 80 and 50 $\mu$M foipan. After washing with the same buffer solution as that used for equilibration, elution was performed with a 20 mM phosphate buffer solution (pH 7.5) containing 2 M potassium thiocyanate, 0.4 M $\alpha$-methylmannoside, 0.01% Tween 80 and 50 $\mu$M foipan. ELISA analysis of the TPA antigen in the liquid which had passed through this ConA-Sepharose column, the washing solution and the eluate solution revealed that in every case the TPA derivative under investigation had been adsorbed by the ConA-Sepharose column and was recovered in the eluate solution.

Next, the solution eluted from the ConA-Sepharose column was dialyzed against a 20 mM phosphate buffer solution (pH 7.5) containing 0.15 M sodium chloride, 0.01% Tween 80 and 50 $\mu$M foipan, and then charged into an antibody column ESP-2 (Bioscott Inc.) immobilized Sepharose equilibrated with the same buffer solution. After washing the column with a 20 mM phosphate buffer solution (pH 7.5) containing 0.3 M potassium thiocyanate, 0.15 M sodium chloride and 0.01% Tween 80, elution was performed with a 20 mM phosphate buffer solution (pH 7.5) containing 3 M potassium thiocyanate, 0.15 M sodium chloride and 0.01% Tween 80. The amount of protein contained in this eluate solution was measured by the Lowry method using bovine serum albumine as a standard. The results demonstrated that approximately 1-3 mg of each TPA derivative was obtained from 2-6 liters of the culture broth after cultivation of the corresponding transformant.

Example 2

Measurement of plasminogen activation ability

The plasminogen activation ability of KM4 and KS48 was compared with that of recombinant TPA obtained from CHO-K1 cells transformed with the native TPA expression vector pSVeCPA-1 (Japanese Laid-Open Patent Publication No. 62-14783). The measurements were performed by the following procedure, in accordance with the method of Takada et al., employing the synthetic substrate S-2251 on which plasmin acts [Takada, A., et al., Haemostasis, 18, 117 (1988)]. The TPA was used in the reaction after conversion to two-chain form, and the plasminogen used was human lysine type plasminogen (American Diagnostica).

Lysine type plasminogen was added at concentrations in the range of 11-88 nM to aliquots of 20 mM phosphate buffer solution (pH 7.5) containing 0.1 M sodium chloride, 0.05% Tween 80, 0.3 mM S-2251, and 0.1 mg/ml cyanogen bromide-treated human fibrinogen. The aforesaid TPA derivatives (KM4 or KS48) were added to aliquots of this solution in amounts so that the concentrations of the respective TPA derivatives would be 0.15 nM and thus, the reaction was initiated. The reaction was conducted at 25°C, and the absorbance at 405 nm was measured at predetermined times. The rate of plasmin generation was determined from the obtained data. A Lineweaver-Burk graph was plotted using this plasmin generation rate, and the parameters Km and Kcat were calculated. The results so obtained are shown in Table 6. The data in this Table show that each of the tested TPA derivatives of the present invention displayed higher plasminogen activation ability than recombinant TPA obtained from CHO-K1 cells.

EP 0 420 502 B1

Table 6

| TPA derivative | Km(nM) | $V_{max}$(nM·min) | Kcat(sec$^{-1}$) | Kcat/Km(nM·sec$^{-1}$) |
|---|---|---|---|---|
| Native TPA | 41.5 | 0.784 | 0.0871 | 0.00210 |
| KM4 | 29.2 | 1.92 | 0.213 | 0.00729 |
| KS48 | 32.8 | 0.72 | 0.0800 | 0.00244 |

Example 3

Measurement of in vitro thrombolytic ability

Ability of the TPA derivatives obtained in Example 1 (SD8, SD4 and KS48) for the lysis of plasma clot was measured in accordance with the method of Collen, D. et al. [Thromb. Haemost., 52, 308 (1984)], using the following procedure.

First, plasma was prepared from the blood of five volunteers using a citrate anticoagulant, then these plasmas were mixed and used for the following measurements. To 1 ml aliquots of plasma were added 10 $\mu$l of $^{125}$I-labelled fibrinogen (10 MBq/ml), 25 $\mu$l of human $\alpha$-thrombin (100 units/ml) and 50 $\mu$l of 0.5 M calcium chloride, then the mixture was promptly placed in a silicone tube (inner diameter 4 mm, outer diameter 8 mm) and allowed to coagulate for 1 hour at 37°C. The silicone tube containing the clot so formed was cut into 1 cm lengths, after which the clots were removed from the tube and washed with 0.85% sodium chloride solution.

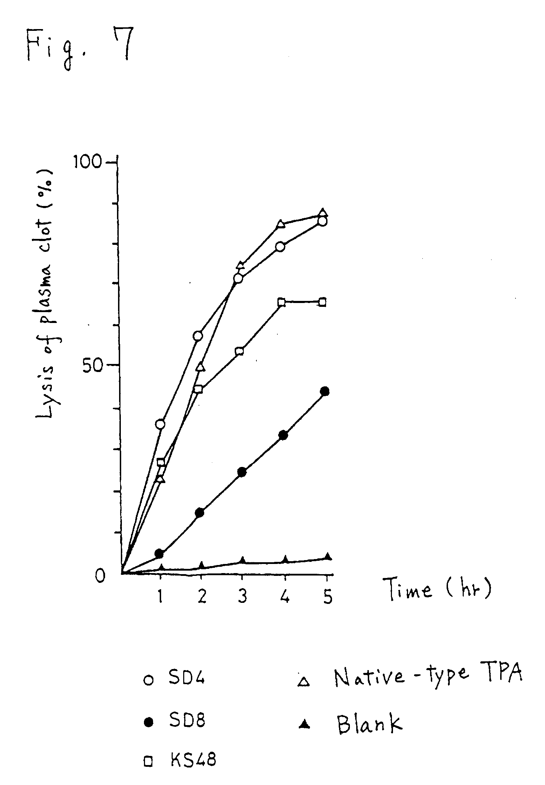

Each of the plasma clots prepared in this manner was floated in 2.5 ml of plasma, then the TPA or TPA derivative to be tested was added, allowing the reaction to be initiated. After the commencement of the reaction, 50 $\mu$l of the plasma was sampled every hour, and the radioactivity of the $^{125}$I which had been released from the plasma clot into the plasma was measured with a gamma-counter. This measurement was continued over a 5-hour period. Taking the radioactivity released by complete dissolution of the clot as 100%, the percentage of dissolution for each sampling time was determined. The results so obtained are shown in Figure 7. Recombinant TPA obtained from CHO-K1 cells transformed with the native TPA expression vector pSVePA-1 (Japanese Laid-Open Patent Publication No. 62-14783) was used as a control. SD4 displayed lytic ability of the same order as CHO recombinant TPA. KS48 also displayed lytic ability of the same level as CHO recombinant TPA up to 2 hours after the commencement of measurement, but subsequently the activity of this derivative tended to diminish. In this figure, the results for the respective analytes are indicated by the symbols ○ for SD4, ● for SD8, □ for KS48, △ for recombinant TPA obtained from CHO-1 cells and ▲ for spontaneous lysis without addition of either TPA or TPA derivatives.

Example 4

Measurement of half-life of TPA derivatives in blood

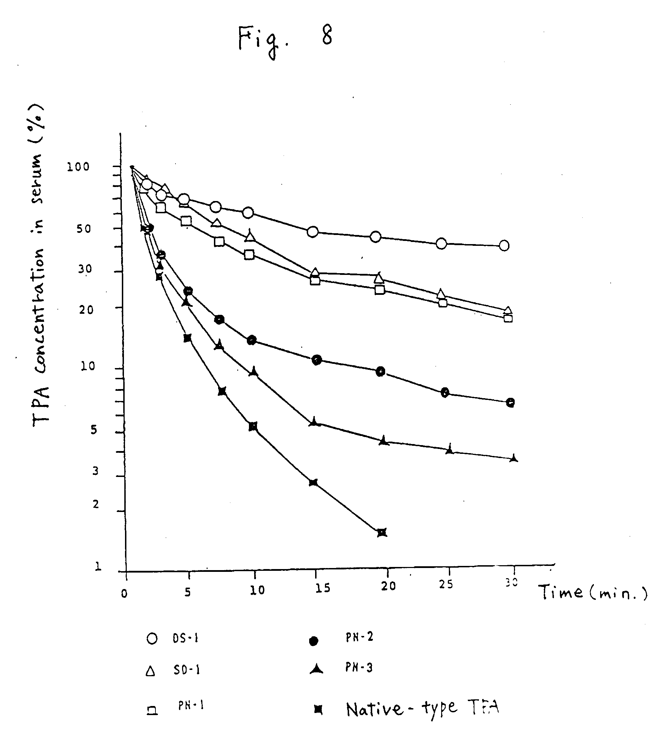

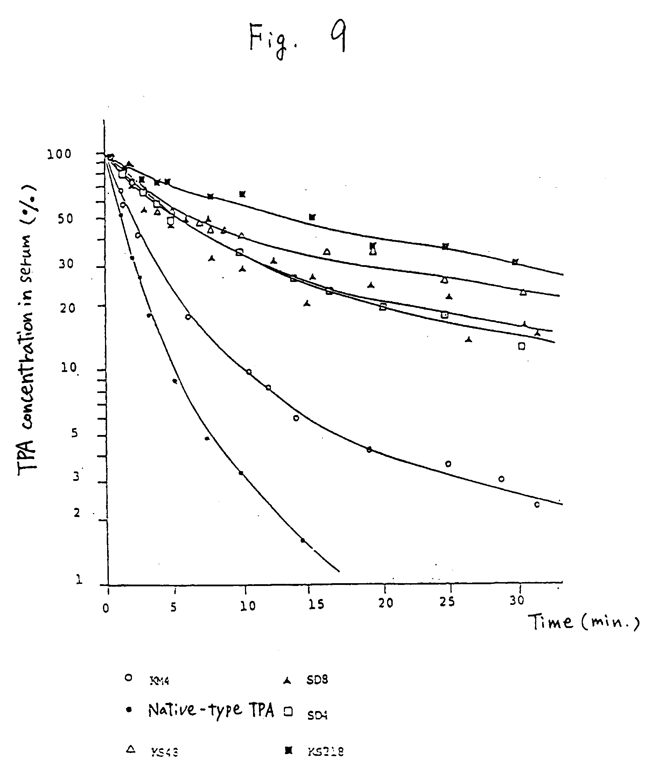

Single doses of approximately 300 $\mu$g of the purified respective TPA derivatives as well as native-type recombinant TPA obtained from CHO-K1 cells (Japanese Laid-Open Patent Publication No. 62-14783, supra) were administered to rabbits via the auricular veins and the half-life of the substance in the bloodstream was measured. Measurement was performed by quantitation of TPA antigen levels in the blood, using an ELISA technique, at the times indicated in Figure 8 or Figure 9. The measurement results for the TPA derivatives PN1, PN2, PN3, DS1 and SD1 are shown in Figure 8 together with the corresponding results for the recombinant native TPA. In this figure, the results for the respective analytes are indicated by the symbols ○ for DS1, △ for SD1, □ for PN1, ● for PN2, ▲ for PN3, and ■ for the recombinant native TPA. The measurement results for the TPA derivatives KM4, KS48, SD4, KS218 and SD8 are shown in figure 9 together with the corresponding results for the recombinant native TPA. In this figure, the results for the respective analytes are indicated by the symbols ○ for KM4, ▲ for SD8, □ for SD4, ■ for KS218, △ for KS48, and ● for the recombinant native TPA. The results demonstrate that each of TPA derivatives displayed improved persistence in the blood as compared with recombinant native TPA obtained from CHO-K1 cells. In particular, TPA derivatives with amino acid replacements in the region from the 37th to the 42nd amino acid residue from the N-terminus of the amino acid sequence of TPA (i.e., KS48, KS218, SD4 and SD8) displayed far greater persistence in the blood than the recombinant native TPA.

**Claims**

1. A tissue plasminogen activator derivative, characterized by a greatly extended persistence in blood as compared with native TPA, comprising;
   (i) asparagine at the 37th position being replaced by serine,
   (ii) serine at the 38th position being replaced by valine,
   (iii) glycine at the 39th position being replaced by valine,

(iv) arginine at the 40th position being replaced by glutamic acid or isoleucine,

(v) alanine at the 41st position being replaced by serine, phenylalanine, or valine, and

(vi) glutamine at the 42nd position being replaced by serine,

and / or further comprising;

(vii) glycine at the 161st position being replaced by arginine,

(viii) lysine at the 162nd position being replaced by arginine, and

(ix) serine at the 165th position being replaced by tryptophan,

wherein said position number is determined from the N-terminus of the amino acid sequence of natural tissue plasminogen activator.

2. A tissue plasminogen activator derivative, characterized by a greatly extended persistence in blood as compared with native TPA,

comprising;

(i) asparagine at the 37th position being replaced by serine,

(ii) serine at the 38th position being replaced by valine,

(iii) glycine at the 39th position being replaced by valine,

(iv) arginine at the 40th position being replaced by glutamic acid or isoleucine,

(v) alanine at the 41st position being replaced by serine, phenylalanine, or valine, and

(vi) glutamine at the 42nd position being replaced by serine,

and

(vii) asparagine at the 115th position which is in the kringle 1 domain being replaced by proline,

wherein said position number is determined from the N-terminus of the amino acid sequence of natural tissue plasminogen activator.

3. A DNA sequence encoding the tissue plasminogen activator derivative of claim 1 or 2.

4. A DNA sequence according to claim 3, which is derived from cDNA.

5. A DNA sequence according to claim 3, which is derived from genomic DNA.

6. An expression vector comprising a DNA sequence of claim 3.

7. A transformant which is obtained by introducing an expression vector of claim 6 into a cultured animal cell.

8. A transformant according to claim 7, wherein said cultured animal cell is derived from, CHO cell.

9. A glycosylated tissue plasminogen activator derivative obtained by cultivating a transformant of claim 8.

10. A method for producing a tissue plasminogen activator comprising the steps of:

(a) constructing an expression vector which comprises a DNA sequence of claim 3,

(b) introducing said expression vector into a cultured animal cell, resulting in a transformant,

(c) cultivating said transformant to produce a tissue plasminogen activator derivative, and

(d) recovering the produced tissue plasminogen activator derivative.

**Patentansprüche**

1. Gewebeplasminogenaktivator-Derivat, gekennzeichnet durch eine im Vergleich mit nativem TPA stark verlängerte Persistenz im Blut, umfassend

(i) Asparagin an der Position 37 ist durch Serin ersetzt,

(ii) Serin an der Position 38 ist durch Valin ersetzt,

(iii) Glycin an der Position 39 ist durch Valin ersetzt,

(iv) Arginin an der Position 40 ist durch Glutaminsäure oder Isoleucin ersetzt,

(v) Alanin an der Position 41 ist durch Serin, Phenylalanin oder Valin ersetzt, und

(vi) Glutamin an der Position 42 ist durch Serin ersetzt,

und/oder ferner umfassend

(vii) Glycin an der Position 161 ist durch Arginin ersetzt,

(viii) Lysin an der Position 162 ist durch Arginin ersetzt, und

EP 0 420 502 B1

(ix) Serin an der Position 165 ist durch Tryptophan ersetzt,

wobei die Positionszahl vom N-Terminus der Aminosäuresequenz von natürlichem Gewebeplasminogenaktivator bestimmt ist.

**2.** Gewebeplasminogenaktivator-Derivat, gekennzeichnet durch eine im Vergleich mit nativem TPA stark verlängerte Persistenz im Blut, umfassend

(i) Asparagin an der Position 37 ist durch Serin ersetzt,

(ii) Serin an der Position 38 ist durch Valin ersetzt,

(iii) Glycin an der Position 39 ist durch Valin ersetzt,

(iv) Arginin an der Position 40 ist durch Glutaminsäure oder Isoleucin ersetzt,

(v) Alanin an der Position 41 ist durch Serin, Phenylalanin oder Valin ersetzt, und

(vi) Glutamin an der Position 42 ist durch Serin ersetzt, und

(vii) Asparagin an der Position 115 innerhalb der Kringel-1-Region ist durch Prolin ersetzt,

wobei die Positionszahl vom N-Terminus der Aminosäuresequenz von natürlichem Gewebeplasminogenaktivator bestimmt ist.

**3.** DNA-Sequenz, die für das Gewebeplasminogenaktivator-Derivat gemäß Anspruch 1 oder 2 kodiert.

**4.** DNA-Sequenz nach Anspruch 3, die von cDNA abgeleitet ist.

**5.** DNA-Sequenz nach Anspruch 3, die von genomischer DNA abgeleitet ist.

**6.** Expressionsvektor, welcher eine DNA-Sequenz gemäß Anspruch 3 umfaßt.

**7.** Transformante, die erhalten wird durch Einführung eines Expressionsvektors gemäß Anspruch 6 in eine kultivierte tierische Zelle.

**8.** Transformante nach Anspruch 7, bei der sich die kultivierte tierische Zelle von einer CHO-Zelle ableitet.

**9.** Glykosyliertes Gewebeplasminogenaktivator-Derivat, erhalten durch Kultivierung einer Transformante gemäß Anspruch 8.

**10.** Verfahren zur Herstellung eines Gewebeplasminogenaktivators, umfassend die Schritte

(a) Herstellung eines Expressionsvektors, der eine DNA-Sequenz gemäß Anspruch 3 umfaßt,

(b) Einführung des Expressionsvektors in eine kultivierte tierische Zelle, wodurch eine Transformante resultiert,

(c) Kultivierung der Transformante zur Herstellung eines Gewebeplasminogenaktivator-Derivats, und

(d) Gewinnung des gebildeten Gewebeplasminogenaktivator-Derivats.

**Revendications**

**1.** Dérivé d'activateur de plasminogène de tissu, caractérisé par une persistance fortement prolongée dans le sang par comparaison avec le tPA natif, comprenant,

(i) un remplacement de l'asparagine de la 37ème position par la sérine,

(ii) un remplacement de la sérine de la 38ème position par la valine,

(iii) un remplacement de la glycine de la 39ème position par la valine,

(iv) un remplacement de l'arginine de la 40ème position par l'acide glutamique ou l'isoleucine,

(v) un remplacement de l'alanine à la 41ème position par la sérine, la phénylalanine ou la valine, et

(vi) un remplacement de la glutamine à la 42ème postion par la sérine,

et/ou comprenant en outre:

(vii) un remplacement de la glycine à la 161ème position par l'arginine,

(viii) un remplacement de la lysine à la 162ème position par l'arginine, et

(ix) un remplacement de la sérine à la 165ème position par le tryptophane,

ledit numéro de position étant déterminé à partir de la terminaison N de la séquence d'acides aminés de l'activateur naturel de plasminogène de tissu.

**2.** Dérivé activateur de plasminogène de tissu, caractérisé par une persistance fortement prolongée dans le sang par comparaison avec le tPA natif, comprenant:

28

(i) un remplacement de l'asparagine de la 37ème position par la sérine,

(ii) un remplacement de la sérine de la 38ème position par la valine,

(iii) un remplacement de la glycine de la 39ème position par la valine,

(iv) un remplacement de l'arginine de la 40ème position par l'acide glutamique ou l'isoleucine,

(v) un remplacement de l'alanine à la 41ème position par la sérine, la phénylalanine ou la valine, et

(vi) un remplacement de la glutamine à la 42ème position par la sérine,

et

(vii) un remplacement de l'asparagine à la 115ème position, qui est dans la région kringle 1, par la proline,

ledit numéro de position étant déterminé à partir de la terminaison N de la séquence d'acides aminés de l'activateur naturel de plasminogène de tissu.

3. Séquence d'ADN encodant le dérivé d'activateur de plasminogène de tissu selon la revendication 1 ou 2.

4. Séquence d'ADN selon la revendication 3, qui est dérivée du cADN.

5. Séquence d'ADN selon la revendication 3, qui est dérivée de l'ADN génomique.

6. Vecteur d'expression comprenant une séquence d'ADN selon la revendication 3.

7. Transformant qui est obtenu en introduisant un vecteur d'expression selon la revendication 6 dans une cellule animale cultivée.

8. Transformant selon la revendication 7, dans lequel ladite cellule animale cultivée est dérivée d'une cellule CHO.

9. Dérivé glycosylé d'activateur de plasminogène de tissu obtenu en cultivant un transformant selon la revendication 8.

10. Procédé de production d'un activateur de plasminogène de tissu comprenant les étapes consistant à:

(a) construire un vecteur d'expression qui comprend une séquence d'ADN selon la revendication 3,

(b) introduire ledit vecteur d'expression dans une cellule animale cultivée, d'où il résulte un transformant,

(c) cultiver ledit transformant afin de produire un dérivé d'activateur de plasminogène de tissu et

(d) récupérer le dérivé produit d'activateur de plasminogène de tissu.

Fig. 1

EP 0 420 502 B1

Fig. 2

5' GATCTTACCAAGTGATCTGCAAGAAGAAGAAAACGCAGATGATATACCAGCAACATCAGTCATG 3'

5' ACTGATGTTGCTCGTATATCATCTGCGTTTTCTTCTTCTTGCAGATCACTTGGTAA 3'

Fig. 3

EP 0 420 502 B1

# Fig. 4

BglII-NarI fragment (about 0.33Kb)

⑨ BglII - SspI fragment (about 100bp)

⑩ DraIII - NarI fragment (about 200 bp)

⑪ BglII - NarI fragment (about 8.1 Kb)

T4DNA ligase

Mutation

⑧ Synthetic DNA fragment

5′ GTGGCAGGAAAACTCCACCACAGAGCACCAGCAAT 3′

5′ ATTGCTGGTGCTCTGTGGTGGAGTTTTCCTGCCACTCA 3′

Fig. 5

① BglII - NarI fragment (about 0.33 kb)

T4 DNA ligase

② BglII-NarI fragment (about 8.1 kb)

Fig. 6

Fig. 7

Legend:
- o SD4
- • SD8
- □ KS48
- △ Native-type TPA
- ▲ Blank

Fig. 8

Fig. 9